# EUROPEAN PATENT APPLICATION

(11) **EP 4 609 855 A1**
(43) Date of publication of application: **03.09.2025**
(21) Application number: 23882999.8
(22) Date of filing: 19.10.2023
(51) Int. Cl.: A61K 9/00, A61K 9/14, A61K 31/465

(54) **METHOD FOR MANUFACTURING NICOTINE DRY POWDER FOR INHALATION**

(30) Priority: 27.10.2022 KR 20220140558; 06.10.2023 KR 20230133753
(71) Applicant: KT&G Corporation, Daedeok-gu Daejeon 34337 (KR)
(72) Inventor: JUNG, Yongmi, Daejeon 34128 (KR); KIM, Moonwon, Daejeon 34128 (KR); SEO, Man Seok, Daejeon 34128 (KR); SHIN, Jun Won, Daejeon 34128 (KR)
(74) Representative: Ter Meer Steinmeister & Partner
(86) International application number: PCT/KR2023/016237
(87) International publication number: WO 2024/090892

(57) **Abstract**

The present invention provides a method for manufacturing a nicotine dry powder for inhalation, the method comprising the steps of: (S1) preparing a nicotine powder containing nicotine or a nicotine salt, an amino acid, and an excipient; (S2) preparing a flavor powder containing a flavoring agent and an excipient; and (S3) mixing the nicotine powder and the flavor powder at a weight ratio of 9:1 to 6:4, wherein the content of nicotine is 0.5 wt% to 1.7 wt% relative to the total weight of the nicotine dry powder for inhalation.

## Description

### TECHNICAL FIELD

The present disclosure relates to a method of manufacturing a nicotine dry powder for inhalation.

### BACKGROUND ART

Nicotine is delivered into the body by various methods, such as smoking combustible tobacco such as cigarettes, cigars, or pipes, using a method of generating an aerosol by heating an aerosol-generating substance in a cigarette, or directly inhaling a nicotine powder or the like.

Among these, the method of directly inhaling the nicotine powder uses a dry powder inhaler (DPI) to deliver the dry powder directly into the user's respiratory tract by inhalation. In addition to nicotine, the dry powder is also used to contain various effective ingredients (drugs) for the purpose of improving diseases, and the like.

Meanwhile, a nicotine dry powder for inhalation of the related art is normally manufactured by performing spray drying by mixing all materials for dry powderization. However, this method has the disadvantage that the transfer of the manufactured nicotine dry powder is not easy, and the characteristics of each substance are different, making it somewhat difficult to mix all substances and secure ease of manufacture.

### DISCLOSURE OF THE INVENTION

### TECHNICAL GOALS

One object of the present disclosure is to solve the above problems, and provide a method of manufacturing a nicotine dry powder for inhalation, which includes a nicotine powder and a flavoring powder, and capable of obtaining both appropriate transfer characteristics of nicotine and a flavor by making the nicotine powder into ultrafine particles through spray drying and through a milling process for the flavoring powder.

However, goals to be achieved are not limited to those described above, and other goals not mentioned above are clearly understood by one of ordinary skill in the art from the following description.

### TECHNICAL SOLUTIONS

According to one embodiment of the present disclosure, there is provided a method of manufacturing a nicotine dry powder for inhalation, the method including:
a step S1 of manufacturing a nicotine powder including nicotine or a nicotine salt, amino acid, and an excipient;
a step S2 of manufacturing a flavoring powder including a flavoring agent and an excipient; and
a step S3 of mixing the nicotine powder and the flavoring powder at a weight ratio of 9:1 to 6:4,
wherein a content of the nicotine is 0.5 wt% or more and 1.7 wt% or less based on a total weight of the nicotine dry powder for inhalation.

### EFFECTS OF THE INVENTION

A method of manufacturing a nicotine dry powder for inhalation according to one aspect of the present disclosure may ensure ease of manufacturing compared to a method of manufacturing a nicotine dry powder of the related art, and a nicotine dry powder manufactured through the method has the advantage of not only having excellent transfer characteristics of nicotine and a flavor, but also having a good yield of a nicotine content.

### BRIEF DESCRIPTION OF DRAWINGS

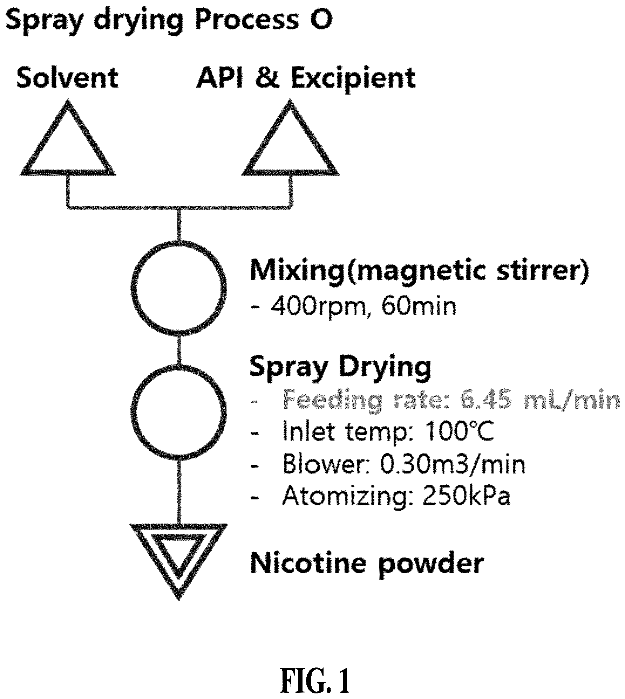

FIG. 1 is a diagram illustrating a manufacturing condition of a nicotine powder manufactured through a spraying process (spray drying) in a manufacturing method according to one embodiment of the present disclosure.

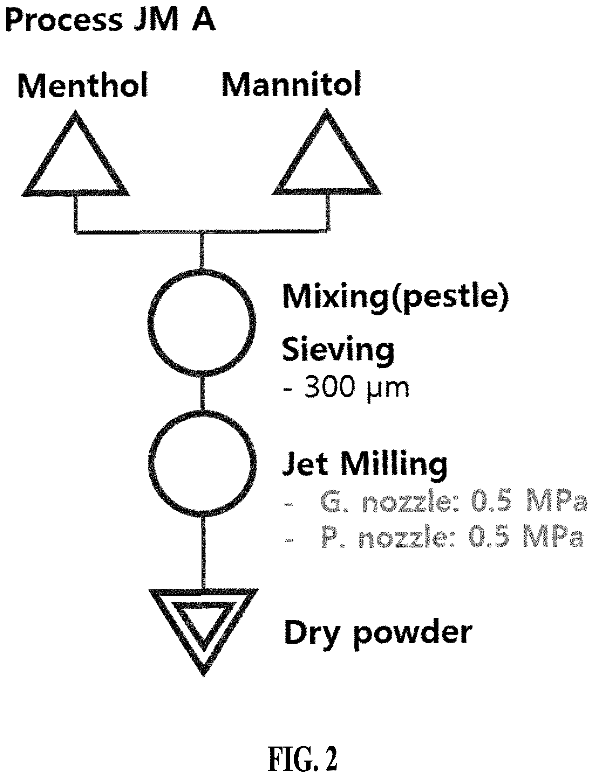

FIG. 2 is a diagram illustrating a manufacturing condition of a flavoring powder manufactured through jet milling in a manufacturing method according to one embodiment of the present disclosure.
FIG. 3 is a diagram illustrating a result of measuring an amount of a nicotine dry powder composition transferred per puff according to one embodiment of the present disclosure.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. The embodiments should be understood to include all modifications, equivalents, and substitutions within the scope and spirit of the present disclosure.

The terminology used herein is for the purpose of describing particular embodiments only and is not to be limiting of the embodiments. As used herein, the singular form is intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, the same or similar elements are denoted by the same reference numerals even though they are depicted in different drawings, and redundant descriptions thereof will be omitted. In the following description of the embodiments, a detailed description of known functions and configurations incorporated herein will be omitted when the same may make the subject matter of the embodiments disclosed in the present specification rather unclear.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless otherwise mentioned, the descriptions of one embodiment may be applicable to other embodiments and thus, repeated descriptions will be omitted for conciseness.

The term "dry powder inhaler (DPI)" used herein refers to a device that delivers substances produced by a reaction between a nicotine-related substance and an organic acid substance into the user's lungs through the user's oral cavity.

According to one embodiment of the present disclosure, there is provided a method of manufacturing a nicotine dry powder for inhalation, the method including:
a step S1 of manufacturing a nicotine powder including nicotine or a nicotine salt, amino acid, and an excipient;
a step S2 of manufacturing a flavoring powder including a flavoring agent and an excipient; and
a step S3 of mixing the nicotine powder and the flavoring powder at a weight ratio of 9:1 to 6:4,
wherein a content of the nicotine is 0.5 wt% or more and 1.7 wt% or less based on a total weight of the nicotine dry powder for inhalation.

Each step of the manufacturing method is described in detail below.

The step S1 is a step of manufacturing a nicotine powder including nicotine or a nicotine salt, the nicotine salt may be present in the form of a salt of nicotine and organic acid, and the organic acid may correspond to, but is not limited to, lactic acid, tartaric acid, pyruvic acid, benzoic acid, citric acid, and/or salicylic acid.

When forming the nicotine salt with the organic acid as described above, the nicotine salt may be in the form of, for example, nicotine lactate, nicotine tartrate, nicotine pyruvate, or nicotine salicylate. Among these, considering the stability and harmfulness to the human body and the like, since the nicotine dry powder is used as the nicotine dry powder for human inhalation, the organic acid is desirably lactic acid, and the nicotine salt is desirably nicotine lactate accordingly.

Meanwhile, when the nicotine salt is nicotine lactate, in the manufacturing process of the nicotine powder, a molar ratio of specific nicotine and lactic acid is an important factor in determining a nicotine yield. Accordingly, from a viewpoint of forming a stable nicotine salt and achieving a higher yield of nicotine, it is desirable that the molar ratio of nicotine to lactic acid in the step S1 is 1:4 or more (e.g., 1:5, 1:6, etc.). In particular, since the step S1 is performed by a high-temperature spray drying process to be described in detail below, it may be seen that the molar ratio of nicotine to lactic acid as described above is more suitable for forming a stable salt as it does not cause volatilization even at a high temperature.

In addition, when the molar ratio of lactic acid provided in the nicotine salt is excessively great (e.g., 1:10), the yield in the nicotine powder may be excellent compared to the nicotine input, but it is not appropriate in terms of the user's inhalation stability, and it is desirably 1:9 or less (e.g., 1:7, 1:8, etc.).

The nicotine powder may contain amino acid and an excipient, in addition to the nicotine or the nicotine salt.

The type of amino acid is not particularly limited, but may include one or more of leucine, lysine, valine, arginine, threonine, phenylalanine, glycine and/or methionine, and the amino acid may desirably include leucine.

As described above, when leucine is included as the amino acid, flowability and a transfer amount of a finally formed nicotine dry powder may be improved, unlike in a case of other amino acids.

In addition, it is desirable that the content of amino acid included at this time is 5 wt% or less based on a total content of the dry powder. By controlling the content of amino acid to 5 wt% or less as described above, a particle size and particle size distribution of the finally formed nicotine dry powder may be improved.

The type of excipient included in the nicotine powder may include sugar and/or sugar alcohol, and the type of sugar may include sucrose, trehalose, dextrose, glucose, maltose, and the like. Furthermore, the type of sugar alcohol may include mannitol, sorbitol, galactitol, maltitol, xylitol, lactitol, and the like.

Among these, it is desirable that the excipient include mannitol, which has an advantage in formulation through spray drying. Specifically, the mannitol is water-soluble, stable in moisture before and after formulation, has no particularly negative sensory characteristics other than a slight sweetness, and has no particular side effects, and accordingly, it is particularly advantageous for use as a carrier material for delivering nicotine. In addition, the mannitol has the advantage of being able to bind to sputum (phlegm) in the bronchial tubes to easily discharge sputum (phlegm) when absorbed into the bronchial tubes.

The nicotine dry powder is manufactured specifically through a spray drying process using the nicotine or the nicotine salt, the amino acid, and the excipient described in detail above. When nicotine powder is manufactured using the spray drying process, better results are obtained in terms of yield than when jet milling is used. This is understood to be because, when jet milling or the like is used, nicotine evaporates into the air due to the characteristics of the nicotine powder.

Meanwhile, when performing the spray drying, a solvent, the nicotine or nicotine salt, the amino acid, and the excipient, as shown in Table 1 below, may be stirred first using a magnetic stirrer, and then the spray drying may be performed.

At this time, water is used as the solvent, and the spray drying is performed at an inlet temperature (inlet temp.) of about 100°C at a specific spray flow rate and atmosphere flow rate to form a nicotine powder. The spray flow rate is a factor that determines a final yield, a total spray time, properties, and the like of the nicotine powder, and may be appropriately 2 to 7 mL/min. The atmosphere flow rate thereof may be appropriately 0.3 to 0.4 m³/min, considering the final yield and the particle size of the nicotine powder. In addition, the flow rate of an aspirator is determined by pressure or a flow rate depending on the characteristics of the equipment, and may be appropriately about 250 kPa or 1200 L/hr.

Thereafter, the step S2 is a step of manufacturing a flavoring powder including a flavoring agent and an excipient. The flavoring agent refers to a flavor commonly used in a smoking article and the like, and may include, for example, rosemary, eucalyptol, licorice, sucrose, fructose syrup, isosweet, cocoa, lavender, cinnamon, cardamom, celery, fenugreek, cascarilla, sandalwood, bergamot, geranium, honey essence, rose oil, vanilla, lemon oil, orange oil, mint oil, mandarin oil, catechin, grapefruit, caraway, cognac, jasmine, chamomile, menthol, cinnamon, ylang-ylang, sage, spearmint, ginger, coriander, and coffee, but is not limited thereto.

Furthermore, by containing a flavoring agent in the flavoring powder, it is possible to not only evoke a sense of flavor for the user, but also exhibit a cough suppressing function, such as expanding the bronchial tubes, in addition to its role as a flavoring agent, optionally depending on the flavoring agent. In this respect, it is desirable that the flavoring agent include menthol.

In addition, as in the nicotine powder described above, the flavoring powder may also contain the excipient, and the type of excipient may include sugar including sucrose, trehalose, dextrose, glucose, or maltose and/or sugar alcohol including mannitol, sorbitol, galactitol, maltitol, xylitol, or lactitol as described in detail above. Among them, it is desirable to use mannitol as the excipient.

The nicotine dry powder for inhalation according to one embodiment of the present disclosure may contain the nicotine powder and the flavoring powder at a weight ratio of 9:1 to 5:5, desirably at a weight ratio of 8:2 to 6:4.

By adjusting the nicotine powder and the flavoring powder within the above range, the particle size, the particle distribution, and the transfer characteristics of the powder are good, and thus, the user may satisfy both the functions of nicotine and flavor.

The step S2 is performed specifically through a jet milling process using the flavoring agent and the excipient described in detail above. When performing the jet milling, it is desirable to lower the temperature so that the temperature does not rise. This is because the flavoring agent such as menthol easily evaporate at a high temperature, which results in a decrease in the content included in the powder or a negative effect on the transfer amount. Therefore, it is desirable to manufacture the flavoring powder in a separate step through low-temperature jet milling, unlike the nicotine powder, which is spray-dried at a high temperature.

Meanwhile, in addition to the jet milling, low-temperature milling and pestle milling may also be used. In particular, the low-temperature milling may be used as a method to prevent agglomeration of powders by lowering the ambient temperature in order to prevent the temperature rise due to strong contact between powders during general milling, and the temperature at this time may be in a range of -20°C to 5°C.

Thereafter, in the step S3, the manufactured nicotine powder and flavoring powder may be mixed (post-mixed) at a weight ratio of 8:2 to 6:4, through which the components such as nicotine contained in the nicotine powder and the flavoring powder may be evenly mixed without loss of the components, thereby showing excellent results in terms of transition characteristics when inhaled by the user.

Meanwhile, after the step S3, a step S4 of additionally mixing a cough suppressant component may be included optionally.

In this case, the nicotine dry powder for inhalation to be finally formed may further include a cough suppressant in addition to the nicotine powder and the flavoring powder. The type of cough suppressant is not particularly limited, but include menthol, mint, saccharin, benzocaine, and the like.

As described above, even when the nicotine dry powder for inhalation includes the cough suppressant component separately, in order to exhibit the effect according to one embodiment of the present disclosure, the nicotine content, the amino acid content, the weight ratio of the nicotine powder to the flavoring powder, and the like need to satisfy the desirable ranges described above.

Hereinafter, the configuration of the present disclosure and effects thereof will be described in more detail through examples and comparative examples. However, the examples are merely intended for the purpose of describing the disclosure in more detail, and thus, the scope of the disclosure is not limited to the examples.

### <Examples>

### 1. Manufacturing Example: Manufacturing of nicotine dry powder for inhalation

### (1) Example 1

### 1) Manufacturing of nicotine powder - Spray drying

The materials were weighed according to the composition ratio in Table 1 below, placed in DI water, and stirred for about 10 minutes. At this time, a mass ratio of water and solid components was 98:2. A nicotine powder was manufactured by spray drying process and under step conditions shown in Table 1 below.

**[Table 1]**

| | | Weight (g) | Weight% |
|---|---|---|---|
| Example 1 | Nicotine | 0.16 | 0.8 |
| | Lactic acid | 0.8 | 4 |
| | Leucine | 1.66 | 8.3 |
| | Mannitol | 17.38 | 86.9 |
| Total | | 20 | 100 |

### 2) Manufacturing flavoring powder - Jet milling

In order to manufacturing a flavoring powder having the composition shown in Table 2 below, large particles were removed by sieving mannitol and menthol, and powders with small particle sizes were mixed and milled in a milling machine. At this time, the milling was performed repeatedly until the desired particle size was reached while measuring the size of the particles with a Mastersizer.

**[Table 2]**

| | | Weight (g) | Weight% |
|---|---|---|---|
| Example 1 | Mannitol | 2 | 50 |
| | Menthol | 2 | 50 |
| Total | | 4 | 100 |

### 3) Manufacturing of final dry powder composition

The manufactured nicotine powder and flavoring powder were mixed at a weight ratio of 5:1 to manufacture a final nicotine dry powder composition.

### (2) Examples 2 to 4 and Comparative Example 1

In each of Examples 2 to 4, a nicotine dry powder for inhalation was manufactured in the same manner as in Example 1 except that the nicotine salt was changed as in Table 3, and in Comparative Example 1, a nicotine dry powder for inhalation was manufactured in the same manner as in Example 1 except that the flavoring powder was not manufactured separately.

**[Table 3]**

| | Nicotine salt | Flavoring agent |
|---|---|---|
| Example 1 | Nicotine lactate | Menthol |
| Example 2 | Nicotine pyruvate | Menthol |
| Example 3 | Nicotine tartrate | Menthol |
| Example 4 | Nicotine salicylate | Menthol |
| Comparative Example 1 | Nicotine lactate | - |

### 2. Test Example 1: Measurement of particle size and particle size distribution

For the nicotine dry powders manufactured according to the method of manufacturing the nicotine dry powder for inhalation described in the above embodiment, a fine particle fraction (FPF) and a mass median aerodynamic diameter (MMAD), which are indicators of inhalation efficiency, were measured, and results thereof were shown in Table 4 below. The above results are values obtained using a next generation impactor (NGI) according to the guidelines for particle size evaluation of pharmaceutical inhalants.

**[Table 4]**

| | Nicotine salt | Flavoring agent | **FPF** (%, mean ± SD) | MMAD (µm, mean ± SD) |
|---|---|---|---|---|
| Example 1 | Nicotine lactate | Menthol | 47.85 ± 0.27 | 5.26 ± 0.12 |
| Comparative Example 1 | Nicotine lactate | - | 41.55 ± 1.13 | 5.77 ± 0.12 |

As shown in Table 4, according to one embodiment of the present disclosure, the FPF of the nicotine dry powder for inhalation manufactured according to one embodiment of the present disclosure is close to 50%, and the MMAD thereof is also 5.5 µm or less. Thus, it is found that the inhalation efficiency is excellent.

### 3. Test Example 2: Evaluation of transfer characteristics

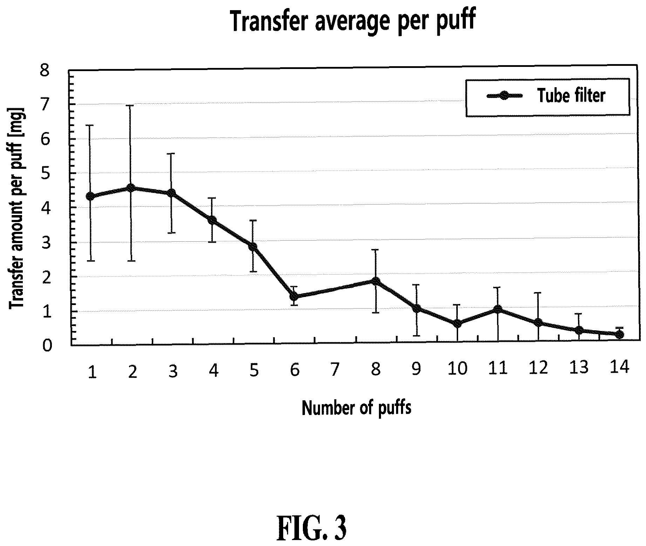

The nicotine dry powder composition of Example 1 manufactured above was applied to a tube filter, and the amount thereof transferred during 14 puffs was shown (FIG. 3).

When the total amount of transfer is 25 mg or more based on 30 mg of filling amount, it may be seen that the total amount of transfer is good. Thus, it may be seen that the nicotine dry powder composition according to one embodiment of the present disclosure has good transfer characteristics.

### 4. Test Example 3: Analysis of nicotine content according to molar ratio of nicotine to lactic acid

In the manufacturing method of Example 1, the molar ratio of nicotine to lactic acid was set to 1:1, 1:2, 1:5, and 1:10, respectively, and spray drying was performed to manufacture a nicotine powder, and the nicotine content in the nicotine powder was measured with respect to an initial input of nicotine.

**[Table 5]**

| Nicotine: lactic acid molar ratio | pH | Nicotine content in nicotine powder after spray drying (based on initial amount of 100%) |
|---|---|---|
| 1:1 | 7.11 | 55.6% |
| 1:2 | 4.03 | 67.85% |
| 1:5 | 3.31 | 83.63% |
| 1:10 | 2.99 | 91.82% |

As shown in Table 5, when the molar ratio of nicotine to lactic acid was 1:5, nicotine content efficiency of 80% or more was shown, and when the molar ratio of nicotine to lactic acid was less than this (e.g., 1:1, 1:2, etc.), the nicotine content efficiency was poor in terms of the yield with respect to the nicotine input. When the molar ratio of nicotine to lactic acid was 1:4 or more, it was found that there is an advantage (yield of about 80% or more) in the manufacturing process of the nicotine powder.

In addition, when the lactic acid content is 1:10, a pH value also decreases significantly, and therefore, it was found that an excessively large amount of lactic acid is also undesirable in terms of inhalation safety.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and equivalents to the claims are also within the scope of the following claims.

## Claims

1. A method of manufacturing a nicotine dry powder for inhalation, the method comprising:
a step S1 of manufacturing a nicotine powder comprising nicotine or a nicotine salt, amino acid, and an excipient;
a step S2 of manufacturing a flavoring powder comprising a flavoring agent and an excipient; and
a step S3 of mixing the nicotine powder and the flavoring powder at a weight ratio of 9:1 to 6:4,
wherein a content of the nicotine is 0.5 wt% or more and 1.7 wt% or less based on a total weight of the nicotine dry powder for inhalation.

2. The method of claim 1, wherein
the nicotine powder comprises a nicotine salt, and
the nicotine salt comprises nicotine, and a salt comprising one or more selected from the group consisting of lactic acid, tartaric acid, pyruvic acid, benzoic acid, citric acid, and salicylic acid.

3. The method of claim 1, wherein the nicotine salt comprises nicotine and lactic acid.

4. The method of claim 3, wherein a molar ratio of the nicotine to lactic acid is 1:4 to 1:9.

5. The method of claim 1, wherein the step S1 is performed by a spray drying process.

6. The method of claim 1, wherein the step S2 is performed by jet milling.

7. The method of claim 1, wherein the amino acid comprises one or more selected from the group consisting of leucine, lysine, valine, arginine, threonine, phenylalanine, glycine, and methionine.

8. The method of claim 1, wherein
the amino acid comprises leucine, and
a content of the leucine is 5 wt% or less based on the total weight of the nicotine dry powder for inhalation.

9. The method of claim 1, wherein
the excipient comprises one or more of sugar and sugar alcohol,
the sugar comprises one or more selected from the group consisting of lactose, sucrose, trehalose, dextrose, glucose, and maltose, and
the sugar alcohol comprises one or more selected from the group consisting of mannitol, sorbitol, galactitol, maltitol, xylitol, and lactitol.

10. The method of claim 1, wherein the excipient comprises mannitol.

11. The method of claim 1, wherein the flavoring agent comprises rosemary, eucalyptol, licorice, sucrose, fructose syrup, isosweet, cocoa, lavender, cinnamon, cardamom, celery, fenugreek, cascarilla, sandalwood, bergamot, geranium, honey essence, rose oil, vanilla, lemon oil, orange oil, mint oil, mandarin oil, catechin, grapefruit, caraway, cognac, jasmine, chamomile, menthol, cinnamon, ylang-ylang, sage, spearmint, ginger, coriander, and coffee.

12. The method of claim 1, further comprising:
additionally mixing a cough suppressant component.

13. The method of claim 12, wherein the cough suppressant component comprises
one or more of menthol, mint, saccharin, and benzocaine.
